(19) Europäisches Patentamt
European Patent Office
Office européen des brevets

(11) **EP 3 048 094 B1**

(12) **EUROPEAN PATENT SPECIFICATION**

(45) Date of publication and mention
of the grant of the patent:
**18.12.2019 Bulletin 2019/51**

(21) Application number: **14845698.1**

(22) Date of filing: **17.09.2014**

(51) Int Cl.:
*C07C 209/68* (2006.01)   *C07C 209/86* (2006.01)
*C07C 211/18* (2006.01)   *C07B 61/00* (2006.01)

(86) International application number:
**PCT/JP2014/074595**

(87) International publication number:
**WO 2015/041261 (26.03.2015 Gazette 2015/12)**

(54) **BIS(AMINOMETHYL)CYCLOHEXANE PRODUCTION METHOD**

HERSTELLUNGSVERFAHREN FÜR BIS(AMINOMETHYL)CYCLOHEXAN

PROCÉDÉ DE PRODUCTION DE BIS(AMINOMÉTHYL)CYCLOHEXANE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.09.2013 JP 2013191881**

(43) Date of publication of application:
**27.07.2016 Bulletin 2016/30**

(73) Proprietor: **Mitsubishi Gas Chemical Company,
Inc.
Tokyo 100-8324 (JP)**

(72) Inventors:
• **YAMAMOTO, Yoshiaki
Niigata-shi
Niigata 950-3112 (JP)**

• **SAMESHIMA, Yuko
Niigata-shi
Niigata 950-3112 (JP)**

(74) Representative: **Hoffmann Eitle
Patent- und Rechtsanwälte PartmbB
Arabellastraße 30
81925 München (DE)**

(56) References cited:
EP-A1- 3 048 095      JP-A- H10 330 329
JP-A- H11 335 335     JP-A- S53 130 637
US-A- 3 344 164       US-A- 3 344 164
US-A- 3 767 707       US-A- 4 086 276

**Description**

Technical Field

The present invention relates to a method for producing bis(aminomethyl)cyclohexane.

Background Art

Bis(aminomethyl)cyclohexane is an industrially important compound to be used as a raw material for e.g., epoxy hardeners, polyamides and polyurethanes. A bis(aminomethyl)cyclohexane has two isomers, i.e., a cis-isomer and a trans-isomer, derived from the cyclohexane ring. It is known that the physical properties of a polymer obtained by using a bis(aminomethyl)cyclohexane greatly vary depending upon the ratio of isomers, i.e., ratio of a cis-isomer and a trans-isomer.

For example, in a polyamide obtained by using 1,4-bis(aminomethyl)cyclohexane, it is known that, as the content of a trans-isomer increases, the melting point of the polyamide increases, with the result that the polyamide becomes highly heat resistant (Non Patent Literature 1). It is also known that a polyurethane obtained by using 1,4-bisisocyanatomethyl cyclohexane derived from 1,4-bis(aminomethyl)cyclohexane is improved in physical properties required for various applications as the content of the trans-isomer increases (Patent Literature 1).

It is further shown that, in a polyamide obtained by using 1,3-bis(aminomethyl)cyclohexane, a polyamide having a high cis-isomer content has a high crystallinity; whereas a polyamide having a high trans-isomer content is amorphous (Non Patent Literature 2).

For these reasons, it is extremely important to control the isomer ratio of a bis(aminomethyl)cyclohexane.

A bis(aminomethyl)cyclohexane is produced by a technique known in the art. More specifically, a bis(aminomethyl)cyclohexane is obtained by hydrogenating an aromatic dinitrile in the presence of a catalyst to synthesize a xylylenediamine and nuclear-hydrogenating the xylylenediamine in the presence of a catalyst.

Many methods are known for producing a xylylenediamine by hydrogenating an aromatic dinitrile. For example, a method of using a Raney catalyst such as a Raney nickel and a Raney cobalt, is disclosed (Patent Literature 3).

Many methods are reported for producing a bis(aminomethyl)cyclohexane by nuclear-hydrogenating a xylylenediamine. For example, a method of using a catalyst such as ruthenium immobilized on a carrier is disclosed (Patent Literature 4)

In the nuclear hydrogenation reaction of a xylylenediamine, a cis-isomer is more easily produced than a trans-isomer, in other words, it is difficult to selectively synthesize a trans-isomer. The ratio of a bis(aminomethyl)cyclohexane trans-isomer produced by this method is generally 50% or less. Because of this, to obtain a 1,4-bis(aminomethyl)cyclohexane having a high trans-isomer content, an isomerization reaction is proposed.

For example, a method for obtaining trans-1,4-bis(aminomethyl)cyclohexane by isomerizing a 1,4-bis(aminomethyl)cyclohexane in the presence of a noble metal catalyst such as platinum and ruthenium is disclosed (Patent Literatures 4 to 6). However, since a content of a trans-isomer produced in one path (single step) remains at about 80%. In order to obtain a trans-isomer in a high-concentration, separation by distillation and crystallization, and recycling are required, in short, a complicated process is required. In this method, isomerization must be carried out in liquid ammonia to obtain 1,4-bis(aminomethyl)cyclohexane in a high recovery rate. Accordingly, this method has a drawback in handling liquid ammonia and being a highpressure reaction. Nevertheless, if liquid ammonia is not used, the recovery rate of 1,4-bis(aminomethyl)cyclohexane decreases.

In the meantime, a method for isomerizing 1,4-bis(aminomethyl)cyclohexane by mixing 1,4-bis(aminomethyl)cyclohexane with a benzylamine compound and an alkali metal, an alkali metal hydride or an alkali metal amide is disclosed (Patent Literature 7). However, it is shown that the final isomer ratio, i.e., a trans/cis ratio, is 4.0 (trans-isomer ratio: about 80%) and isomerization does not proceed any more in this method.

These isomerization reactions have a limitation in that a cis-isomer and a trans-isomer reach an equilibrium state. Thus, it is not easy to obtain 1,4-bis(aminomethyl)cyclohexane containing a trans-isomer more than an equilibrium composition.

In another method (Patent Literature 8) known in the art, trans-1,4-bis(aminomethyl)cyclohexane is obtained by derivatizing 1,4-bis(aminomethyl)cyclohexane to an aldimine compound, and isomerizing and decomposing the aldimine compound.

Also, a method for obtaining a 1,4-bis(aminomethyl)cyclohexane having a high trans-isomer content using terephthalic acid as a raw material via cyclohexane dicarboxylic acid is disclosed (Patent Literature 9). This method discloses that, to increase the content of a trans-isomer, a precursor, 1,4-dicyanocyclohexane, is crystallized to separate a trans-isomer and the remaining cis-isomer is isomerized and recycled. Patent Literature 10 describes a process for obtaining a 1,4-bis(aminomethyl)cyclohexane having a high trans-isomer content wherein isomerization and distillation are carried out simultaneously.

Citation List

Patent Literatures

Patent Literature 1: International Publication No. WO2009/051114

Patent Literature 2: Japanese Patent Laid-Open No. S54-41804

Patent Literature 3: Japanese Patent Laid-Open No. S50-126638

Patent Literature 4: Japanese Patent Laid-Open No. H10-259167

Patent Literature 5: Japanese Patent Laid-Open No. H10-306066

Patent Literature 6: Japanese Patent Laid-Open No. H11-335335

Patent Literature 7: Japanese Patent Publication No. S62-3144

Patent Literature 8: Japanese Patent Laid-Open No. H10-330329

Patent Literature 9: Japanese Patent Laid-Open No. 2011-6382

Patent Literature 10: US 3,344,164

Non Patent Literatures

Non Patent Literature 1: J. Polym. Sci. Part A-1, 10, 465 (1972)
Non Patent Literature 2: KOBUNSHI RONBUNSHU (Japanese Journal of Polymer Science and Technology), Vol. 65, No. 5, pp.305-310 (1979)

Summary of Invention

Technical Problem

In the method described in Patent Literature 8, a trans-isomer is obtained in an extremely high ratio of 99%; however, three steps are required for isomerization; an aldehyde, from which a derivative is obtained, must be recycled through very complicated step. For these reasons, it is not easy to industrially carry out this method.
In the method described in Patent Literature 9, an extremely long step is required and industrially unfavorable.
In the prior art, an industrial technical process for producing 1,4-bis(aminomethyl)cyclohexane having an equilibrium composition (a trans-isomer ratio: 83% or more) in a single step has not yet been established. More specifically, in order to produce a 1,4-bis(aminomethyl)cyclohexane having an equilibrium composition or more (trans-isomer ratio: 83% or more), a step of returning 1,4-bis(aminomethyl)cyclohexane having an equilibrium composition or less (trans-isomer ratio: 83% or less) again to an isomerization step is required, with the result that two steps or more are required.
The present invention was attained in consideration of the aforementioned problems. An object of the present invention is to provide a method for producing a bis(aminomethyl)cyclohexane having a content of an isomer beyond that for an equilibrium composition in a simple process suitable for industrialization.

Solution to Problem

The present inventors intensively conducted studies to solve the aforementioned problems. As a result, they found that the above problems can be solved by a production method of carrying out an isomerization step and a distillation step at the same time, and arrived at the present invention, as defined by the appended claims.
More specifically, the present invention is as follows.

[1] A method for producing a bis(aminomethyl)cyclohexane comprising:

an isomerization step of isomerizing a cis-isomer of 1,4-bis(aminomethyl)cyclohexane and/or a trans-isomer of 1,3-bis(aminomethyl)cyclohexane at a bottom part of a distillation tower to obtain a trans-isomer of 1,4-bis(aminomethyl)cyclohexane and/or a cis-isomer of 1,3-bis(aminomethyl)cyclohexane, in the presence of at least one compound selected from the group consisting of an alkali metal, an alkali metal-containing compound, an alkaline-earth metal and an alkaline-earth metal-containing compound, and a benzylamine compound; and a distillation step of separating the trans-isomer of 1,4-bis(aminomethyl)cyclohexane and/or the cis-isomer of 1,3-bis(aminomethyl)cyclohexane by distillation, in a top part of the distillation tower, wherein a use amount of the at least one compound selected from the group consisting of an alkali metal, an alkali metal-containing compound, an alkaline-earth metal and an alkaline-earth metal-containing compound relative to one equivalent of a benzylamine compound is 1 to 4 molar equivalent; a use amount of the benzylamine compound relative to 100 wt% of bis(aminomethyl)cyclohexane is 0.50 to 4.0 wt%; an isomerization reaction temperature of the isomerization step is 80 to 140°C; and the isomerization step and the distillation step are simultaneously carried out.

[2] The method for producing the bis(aminomethyl)cyclohexane according to the above [1], wherein the distillation step is carried out to separate a trans-isomer of 1,4-bis (aminomethyl)cyclohexane by distillation such that a content of the trans-isomer in the 1,4-bis(aminomethyl)cyclohexane obtained from the top part of the tower in the distillation step is 84% or more.

[3] The method for producing the bis(aminomethyl)cyclohexane according to the above [1] or [2], wherein the distillation step is carried out to separate a trans-isomer of 1,4-bis (aminomethyl)cyclohexane by distillation such that the content of the trans-isomer in the 1,4-bis(aminomethyl)cyclohexane obtained from the top part of the tower in the distillation step is 90% or more.

[4] The method for producing the bis(aminomethyl)cyclohexane according to the above [1], wherein the benzylamine compound is at least one selected from the group consisting of benzylamine, 3-methylbenzylamine, 4-methylbenzylamine, dibenzylamine, metaxylylenediamine and paraxylylenediamine.

[5] The method for producing the bis(aminomethyl)cyclohexane according to the above [1] or [4], wherein the alkali metal comprises metallic sodium.

[6] The method for producing the bis(aminomethyl)cyclohexane according to any one of the above [1] to [5], wherein the alkali metal-containing compound comprises at least one selected from the group consisting of an alkali metal hydride and an alkali metal amide.

[7] The method for producing the bis(aminomethyl)cyclohexane according to the above [6], wherein the alkali metal hydride comprises sodium hydride.

[8] The method for producing the bis(aminomethyl)cyclohexane according to the above [6], wherein the alkali metal amide comprises sodium amide.

Advantageous Effects of Invention

According to this invention, it is possible to provide a method for producing a bis(aminomethyl)cyclohexane having an isomer content beyond an equilibrium composition in a simple process suitable for industrialization.

Brief Description of Drawings

[Figure 1] Fig.1 shows a schematic view showing an apparatus for conducting the method for producing a bis(aminomethyl)cyclohexane according to the present embodiment.
[Figure 2] Fig.2 shows a graph prepared by plotting distillation data of Comparative Example 2.
[Figure 3] Fig.3 shows a graph prepared by plotting distillation data of Example 1.
[Figure 4] Fig.4 shows a graph prepared by plotting distillation data of Example 2.

Description of Embodiments

Now, embodiments (hereinafter referred to as "the present embodiment") for carrying out the invention will be more specifically described below; however, the present invention is not limited to these and can be modified without departing from the scope of the invention as defined by the appended claims.

[Method for producing a bis(aminomethyl)cyclohexane]

The method for producing a bis(aminomethyl)cyclohexane according to the present embodiment has an isomerization step of isomerizing a cis-isomer of 1,4-bis(aminomethyl)cyclohexane and/or a trans-isomer of 1,3-bis(aminomethyl)cyclohexane at the bottom part of a distillation tower to obtain a trans-isomer of 1,4-bis(aminomethyl)cyclohexane and/or a cis-isomer of 1,3-bis(aminomethyl)cyclohexane, in the presence of at least one compound selected from the group consisting of an alkali metal, an alkali metal-containing compound, an alkaline-earth metal and an alkaline-earth metal-containing compound, and a benzylamine compound; and a distillation step of separating the trans-isomer of 1,4-bis(aminomethyl)cyclohexane and/or the cis-isomer of 1,3-bis(aminomethyl)cyclohexane by distillation, in the top of the distillation tower, wherein

a use amount of the at least one compound selected from the group consisting of an alkali metal, an alkali metal-containing compound, an alkaline-earth metal and an alkaline-earth metal-containing compound relative to one equivalent of a benzylamine compound is 1 to 4 molar equivalent;

a use amount of the benzylamine compound relative to 100 wt% of bis(aminomethyl)cyclohexane is 0.50 to 4.0 wt%;

in which

the isomerization reaction temperature in the isomerization step is 80 to 140°C, and

the isomerization step and the distillation step are simultaneously carried out.

In the method for producing a bis(aminomethyl)cyclohexane according to the present embodiment, if the isomerization step and the distillation step are simultaneously carried out, a 1,4-bis(aminomethyl)cyclohexane having a content of a trans-isomer exceeding that for an equilibrium composition or 1,3-bis(aminomethyl)cyclohexane having a content of a cis-isomer exceeding that for an equilibrium composition can be produced in a simple process.

The term "simultaneous" used herein means not only the case where the initiation and termination points of the isomerization step completely coincide with those of the distillation step but also the case where the isomerization step and the distillation step are partially overlapped; more specifically, refers to the case where a bis(aminomethyl)cyclohexane is separated while isomerizing.

More specifically, if the production method of the present embodiment is carried out in a batch system, the following method may be mentioned: raw materials are supplied to the bottom part of a distillation tower and an isomerization reaction was carried out at the bottom part of the tower; at the same time, a desired isomer is separated by distillation and obtained from the top part of the distillation tower. In contrast, if the production method of the present embodiment is carried out in a continuous system, the following method may be mentioned: raw materials are continuously supplied to the bottom part of a distillation tower and an isomerization reaction is carried out at the bottom part of the tower; at the same time, a desired isomer is continuously separated by distillation and obtained from the top part of the distillation tower. Among them, the continuous system is preferable in view of industrialization.

[Distillation tower]

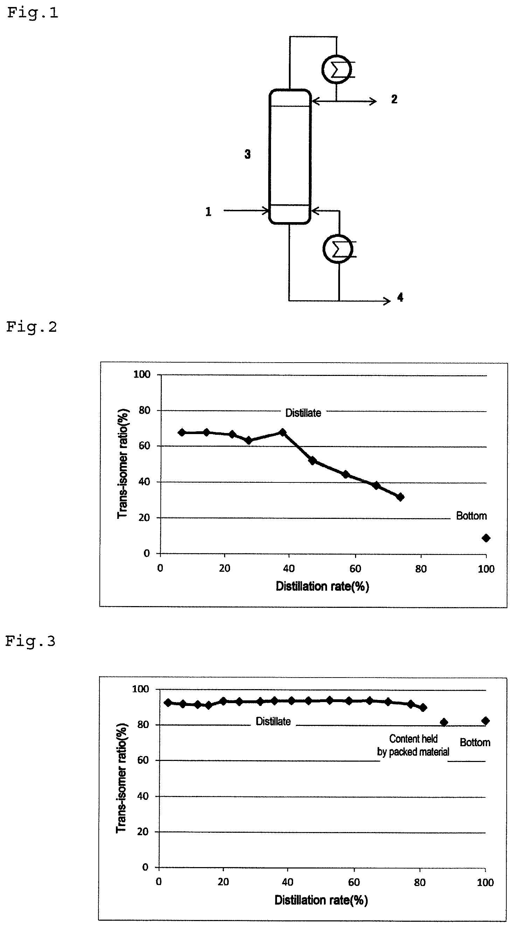

A schematic view of an apparatus for producing a bis(aminomethyl)cyclohexane according to the present embodiment is shown in Figure 1. The apparatus has a distillation tower 3, a supply pipe 1 provided to the bottom part of the tower, a first discharge pipe 2 provided to the top part and a second discharge pipe 4 provided to the bottom part. A mixture of a cis-isomer and a trans-isomer of a bis(aminomethyl)cyclohexane is supplied to the distillation tower 3 through the supply pipe 1. The bis(aminomethyl)cyclohexane separated by distillation is discharged through the first discharge pipe 2 and high boiling-point compounds separated by distillation are discharged from the second discharge pipe 4.

The distillation tower is not particularly limited as long as a cis-isomer and a trans-isomer can be separated and may have a structure known in the art. For example, a packed tower charged with regular packing or irregular packing and a tray tower having trays can be used. Among them, a packed tower charged with regular packing, in which differential pressure is most unlikely produced, is preferably used in order to prevent a temperature increase during an isomerization reaction. The distillation tower may have e.g., a heater for heating a mixture of a cis-isomer and a trans-isomer of a bis(aminomethyl)cyclohexane present at the bottom part, a stirrer for stirring the mixture and a pressure control mechanism for controlling reaction pressure.

[Isomerization step]

The isomerization step is a step of isomerizing a cis-isomer of 1,4-bis(aminomethyl)cyclohexane and/or a trans-isomer of 1,3-bis(aminomethyl)cyclohexane at the bottom part of a distillation tower to obtain a trans-isomer of 1,4-bis(aminomethyl)cyclohexane and/or a cis-isomer of 1,3-bis(aminomethyl)cyclohexane.

The term "isomerize" refers to converting a trans-isomer of 1,3-bis(aminomethyl)cyclohexane to a cis-isomer thereof or a cis-isomer of 1,4-bis(aminomethyl)cyclohexane to a trans-isomer thereof.

In the production method of the present embodiment, for example, in the isomerization step, a mixture of a cis-isomer and a trans-isomer of 1,4-bis(aminomethyl)cyclohexane is subjected to an isomerization reaction to isomerize the cis-isomer of 1,4-bis(aminomethyl)cyclohexane to a trans-isomer of 1,4-bis(aminomethyl)cyclohexane; and a mixture of a cis-isomer and a trans-isomer of 1,3-bis(aminomethyl)cyclohexane is subjected to an isomerization reaction to isomerize the trans-isomer of 1,3-bis(aminomethyl)cyclohexane to a cis-isomer of 1,3-bis(aminomethyl)cyclohexane.

In the isomerization step, the isomerization reaction temperature (tower-bottom temperature) is 80 to 140°C and preferably 100 to 140°C. If the isomerization reaction temperature is 80°C or more, the isomerization reaction can be more efficiently carried out. In contrast, if the isomerization reaction temperature is 140°C or less, a side reaction such as a decomposition reaction of a bis(aminomethyl)cyclohexane can be suppressed, production of side products such as low-boiling products and high-boiling products can be reduced. As a result, a desired isomer of a bis(aminomethyl)cyclohexane can be easily and continuously distilled to improve a yield thereof.

The isomerization reaction can be carried out either in the presence or absence of a solvent. As the solvent that can be used, although it is not particularly limited, for example, an inert solvent is mentioned. Examples of such a solvent include, but are not particularly limited to, aromatic solvents such as benzene, toluene or xylene; ether solvents such as diethyl ether or tetrahydrofuran; and hydrocarbon solvents such as hexane or heptane.

As the isomerization reaction atmosphere, although it is not particularly limited, for example, an atmosphere not containing air or active hydrogen such as water or an alcohol, is preferable. If such an atmosphere is employed, the reaction efficiency tends to be more improved. Particularly, in view of the reaction efficiency, the water content in the reaction system is preferably controlled to be 1000 ppm or less. As a simple method for reducing the content of water in a reaction system, an isomerization reaction is preferably carried out in an atmosphere of an inert gas such as nitrogen gas and argon gas.

[Bis(aminomethyl)cyclohexane]

In the method for producing a bis(aminomethyl)cyclohexane according to the present embodiment, 1,3-bis(aminomethyl)cyclohexane and/or 1,4-bis(aminomethyl)cyclohexane are used. Among them, in view of the effect of the present invention, 1,4-bis(aminomethyl)cyclohexane is preferable. Note that 1,3-bis(aminomethyl)cyclohexane and 1,4-bis(aminomethyl)cyclohexane each may be a mixture of a trans-isomer and a cis-isomer.

As a method for producing a cis-isomer and a trans-isomer of a bis(aminomethyl)cyclohexane or a mixture thereof used in this embodiment, although it is not limited, for example, a method of nuclear-hydrogenating para-xylylenediamine or terephthalonitrile in the presence of a noble metal catalyst such as ruthenium, palladium, rhodium and platinum, is mentioned. The trans-isomer herein is obtained in a ratio of 50% or less and the resultant bis(aminomethyl)cyclohexane or mixture can be used without particularly changing an isomer ratio.

In the isomerization step, a cis-isomer of the 1,4-bis(aminomethyl)cyclohexane and/or a trans-isomer of 1,3-bis(aminomethyl)cyclohexane are isomerized in the presence of at least one compound selected from the group consisting of an alkali metal, an alkali metal-containing compound, an alkaline earth metal and an alkaline earth metal-containing compound (hereinafter collectively referred to as an "alkali metal(s)") and a benzylamine compound. Owing to this, the rate of isomerization is further improved and a recovery rate tends to be more improved.

(Benzylamine compound)

Example of the benzylamine compound include, but are not limited to, for example, monobenzylamine compounds such as benzylamine, 2-methylbenzylamine, 3-methylbenzylamine, 4-methylbenzylamine; secondary benzylamine compounds such as dibenzylamine and N-methylbenzylamine; and compounds having two aminomethyl groups such as metaxylylenediamine and paraxylylenediamine. Among them, in view of reaction efficiency, at least one selected from the group consisting of benzylamine, 3-methylbenzylamine, 4-methylbenzylamine, dibenzylamine, metaxylylenediamine and paraxylylenediamine, is preferable. These compounds may be used alone or in combination of two or more.

The use amount of benzylamine compound relative to bis(aminomethyl)cyclohexane (100 wt%) is 0.50 to 4.0 wt%. If the use amount of benzylamine compound falls within the above range, an isomerization reaction tends to more efficiently proceed.

(Compound)

The compound that is used in the isomerization step is at least one compound selected from the group consisting of an alkali metal, an alkali metal-containing compound, an alkaline-earth metal, and an alkaline-earth metal containing compound. If such a compound is used, an isomerization reaction can more efficiently proceed. These compounds may be used alone or in combination of two or more.

Among them, at least one compound selected from the group consisting of a metallic sodium, a sodium amide and a sodium hydride is preferably included. If such a compound is used, the ratio of a trans-isomer or a cis-isomer in the resultant isomers and the isomerization yield tend to be improved.

Examples of the alkali metals include, but are not particularly limited to, a metallic sodium, a metallic potassium and a metallic lithium.

Examples of the alkali metal-containing compounds include, but are not particularly limited to, an alkali metal hydride, an alkali metal amide and a basic oxide. If such a compound is used, the ratio of a trans-isomer or a cis-isomer in the resultant isomers and the isomerization yield tend to be improved. Among them, at least one selected from the group consisting of an alkali metal hydride and an alkali metal amide, is preferable. Examples of the alkali metal hydride herein include, but are not particularly limited to, sodium hydride, potassium hydride, lithium hydride, lithium aluminum hydride and sodium boron hydride. Examples of the alkali metal amide include, but are not particularly limited to, sodium amide, potassium amide, lithium amide, lithium diisopropylamide and sodium bis(trimethylsilyl)amide. Examples of the basic oxide include, but are not particularly limited to, lithium oxide, sodium oxide, potassium oxide, cesium oxide, magnesium oxide, calcium oxide, strontium oxide and barium oxide.

Examples of the alkaline-earth metal include, but are not particularly limited to, metallic magnesium and metallic calcium.

Examples of the alkaline-earth metal-containing compound include, but are not particularly limited to, an alkaline earth metal hydride. Examples of the alkaline earth metal hydride include, but are not particularly limited to, calcium hydride and magnesium hydride.

The use amount of alkali metal or the like relative to one equivalent of a benzylamine compound is 1 to 4 molar equivalent. Since the use amount of alkali metal or the like falls within the above range, an isomerization reaction tends to more successfully and efficiently proceed.

[Distillation step]

The distillation step is a step of separating a trans-isomer of 1,4-bis(aminomethyl)cyclohexane and/or a cis-isomer of 1,3-bis(aminomethyl)cyclohexane by distillation, in the top part of the distillation tower. In the distillation step, not only a trans-isomer of 1,4-bis(aminomethyl)cyclohexane and/or a cis-isomer of 1,3-bis(aminomethyl)cyclohexane obtained by the above isomerization step but also a trans-isomer of 1,4-bis(aminomethyl)cyclohexane and/or a cis-isomer of 1,3-bis(aminomethyl)cyclohexane present in raw materials in the above isomerization step can be distilled.

In the distillation step, the content of a trans-isomer in 1,4-bis(aminomethyl)cyclohexane obtained from the tower top part is preferably 84% or more and more preferably 90% or more. Note that the term "%" used herein refers to mol%.

Isomerization must be carried out at a tower-bottom temperature within the range of 80 to 140°C and more preferably 100 to 140°C. If the isomerization reaction temperature is 80°C or more, the isomerization reaction tends to proceed more efficiently. In contrast, if the tower-bottom temperature exceeds 140°C, the boiling point increases and the recovery rate of distillation decreases.

Examples

Now, the present invention will be more specifically described by way of Examples and Comparative Examples; however, the present invention is not limited to these Examples.

[Isomer composition]

Isomer compositions (cis/trans ratio) were analyzed by a gas chromatographic apparatus equipped with a capillary column, CP-Volamine manufactured by Valian. The trans-isomer of 1,4-bis(aminomethyl)cyclohexane has a lower boiling point than the cis-isomer thereof. The isomer first detected by gas chromatography was the trans-isomer and the cis-isomer was detected thereafter. The cis-isomer of 1,3-bis(aminomethyl)cyclohexane has a lower boiling point than the trans-isomer thereof. The isomer first detected by gas chromatography is the cis-isomer and the trans-isomer was detected thereafter. The ratio of the trans-isomer was calculated in accordance with the expression:

```
Area value of trans-isomer/(area value for cis-isomer + area value for trans-isomer) × 100.
```

The ratio of the cis-isomer was calculated by the ratio to the trans-isomer from 100.

Recovery rates were obtained by calculating the weight of a bis(aminomethyl)cyclohexane in accordance with the internal standard method of the above gas chromatography analysis, and then, in accordance with the following expression.

$$\text{Recovery rate (\%)} = \text{(bis(aminomethyl)cyclohexane in}$$
$$\text{distillate + bis(aminomethyl)cyclohexane held in packed}$$
$$\text{tower + bis(aminomethyl)cyclohexane in bottom}$$
$$\text{part)/(starting bis(aminomethyl)cyclohexane)} \times 100$$

[Distillation rate]

The distillation rate was calculated in accordance with the following expression.

$$\text{Distillation rate (\%)} = \text{bis(aminomethyl)cyclohexane}$$
$$\text{in distillate/starting bis(aminomethyl)cyclohexane} \times 100$$

[Raw materials]

1,4-Bis(aminomethyl)cyclohexane having an isomer composition (cis/trance ratio) of 59.3/40.7 used herein was obtained by nuclear-hydrogenation of paraxylylenediamine in the presence of a catalyst of Ru-alumina and in accordance with a known technique (for example, Patent Literature 2) and purified by distillation.

[Distillation tower]

As the distillation tower, the tower shown in Figure 1 was used. More specifically, raw materials were supplied batch-wise to the bottom part of the tower and a desired isomer was separated by distillation in the top part of the tower.

<Comparative Example 1>

To 1,4-bis(aminomethyl)cyclohexane (6 g) having an isomer composition (cis/trance ratio) of 59.3/40.7, 4-methylbenzylamine (4-MBA)(0.2 g) and sodium amide (0.2 g) were added. The isomerization reaction was carried out under an argon atmosphere at 120°C and for 4 hours. Note that distillation was not carried out. The isomer composition after the isomerization (cis/trance ratio) was 17/83 and the recovery rate was 96.3%. The isomerization reaction was continued for further two hours; however, the isomer ratio did not change. From this, it is consider that the isomer composition reached an equilibrium.

<Comparative Example 2>

1,4-Bis(aminomethyl)cyclohexane (107 g) having an isomer composition (cis/trance ratio) of 59.3/40.7 was weighed and placed in a distillation tower (theoretical stages: 7) packed with Sulzer pack having an inner diameter of 25 mm, and distillated in the following conditions. The maximum ratio of the trans-isomer of 1,4-bis(aminomethyl)cyclohexane obtained was 67.5%. The trans-isomer ratio decreased as the distillation rate increased. The trans-isomer ratio changed with the distillation rate is shown in Figure 2.

(Conditions)

Tower-bottom temperature: 104 to 113°C
Tower-top pressure: 0.31 to 0.60 kPa (2.3 to 4.5 mmHg)
Tower-bottom pressure: 0.47 to 0.71 kPa (3.5 to 5.3 mmHg)
Reflux ratio: 60 to 120

<Example 1>

In the bottom part of a distillation tower (theoretical stages: 7) packed with Sulzer pack having an inner diameter of 25 mm, 1,4-bis(aminomethyl)cyclohexane (201 g) having an isomer composition (cis/trance ratio) of 59.3/40.7, 4.2 g of 4-methylbenzylamine (4-MBA) and sodium amide (1.6 g) were placed. Ten hours later, distillation and isomerization reaction were performed in the following conditions.

(Conditions)

    Tower-bottom temperature: 104 to 113°C
    Tower-top pressure: 0.31 to 0.60 kPa (2.3 to 4.5 mmHg)
    Tower-bottom pressure: 0.47 to 0.71 kPa (3.5 to 5.3 mmHg)
    Reflux ratio: 60 to 120

Even if the distillation rate increased, the ratio of a trans-isomer did not decrease and 90% or more of 1,4-bis(aminomethyl)cyclohexane was stably obtained. In total, 89% of the distillate was recovered and an average isomer composition (cis/trance ratio) was 8/92. Furthermore, the recovery rate of 1,4-bis(aminomethyl)cyclohexane including that in the bottom liquid was 93% and the isomer composition (cis/trance ratio) was 9/91. If the method of the invention is used, it is possible to obtain 1,4-bis(aminomethyl)cyclohexane having a trans-isomer ratio exceeding the equilibrium composition (isomer composition (cis/trance ratio):17/83). 1,4-Bis(aminomethyl)cyclohexane was obtained in a high recovery rate. The trans-isomer ratio changed with the distillation rate is shown in Figure 3.

<Example 2 (Comparative Example)>

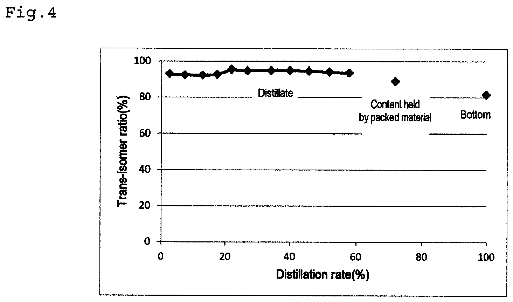

The isomerization reaction and distillation were carried out in the same manner as in Example 1 except that 1,4-bis(aminomethyl)cyclohexane (150 g) having an isomer composition (cis/trance ratio) of 59.3/40.7, 6.2 g of 4-methylbenzylamine (4-MBA) and sodium amide (6.2 g) were used. In total, 70% of distillate was recovered. The average isomer composition (cis/trance ratio) was 7.1/92.9. The recovery rate of 1,4-bis(aminomethyl)cyclohexane including that in the bottom liquid was 90.6% and the isomer composition (cis/trance ratio) was 9.2/90.8. The trans-isomer ratio changed with the distillation rate is shown in Figure 4.

<Comparative Example 3>

To 1,4-bis(aminomethyl)cyclohexane (200 g) having an isomer composition (cis/trance ratio) of 59.3/40.7, 8 g of 4-methylbenzylamine (4-MBA) and sodium amide (8 g) were added, and an isomerization reaction was carried out under an argon atmosphere at 120°C for 5 hours.

After the isomerization reaction, the reaction solution was placed in the bottom of Oldershaw (theoretical stages: 20) distillation tower. While an isomerization reaction was carried out at the bottom part of the distillation tower, distillation was carried out in the following conditions. When the distillation rate exceeded 28%, abnormal bubbling of the tower-bottom liquid and an increase of viscosity occurred, which made it difficult to continue distillation. As a result of analysis of the tower-bottom liquid, high-boiling components produced was 60% or more. The recovery rate of 1,4-bis(aminomethyl)cyclohexane including that in the bottom liquid was up to 42%.

(Conditions)

    Tower-bottom temperature: 150 to 165°C

    Tower-top pressure: 2.00 kPa (15 mmHg)

    Tower-bottom pressure: 4.93 to 5.20 kPa (37 to 39 mmHg)

    Reflux ratio: 120

The present application was made based on Japanese Patent Application No. 2013-191881 filed on September 17, 2013 with Japan Patent Office.

Industrial Applicability

The present invention has industrial applicability as a method for producing a bis(aminomethyl)cyclohexane effective as an optical material using a polyamide and a polyurethane, such as plastic lenses, prisms, optical fibers, information recording substrates and filters. Reference Signs List

1...     Supply pipe
2...     First discharge pipe
3...     Distillation tower
4...     Second discharge pipe

**Claims**

1. A method for producing a bis(aminomethyl)cyclohexane comprising:

    an isomerization step of isomerizing a cis-isomer of 1,4-bis(aminomethyl)cyclohexane and/or a trans-isomer of 1,3-bis(aminomethyl)cyclohexane at a bottom part of a distillation tower to obtain a trans-isomer of 1,4-bis(aminomethyl)cyclohexane and/or a cis-isomer of 1,3-bis(aminomethyl)cyclohexane, in the presence of at least one compound selected from the group consisting of an alkali metal, an alkali metal-containing compound, an alkaline-earth metal and an alkaline-earth metal-containing compound, and a benzylamine compound; and
    a distillation step of separating the trans-isomer of 1,4-bis(aminomethyl)cyclohexane and/or the cis-isomer of 1,3-bis(aminomethyl)cyclohexane by distillation, in a top part of the distillation tower, wherein
    a use amount of the at least one compound selected from the group consisting of an alkali metal, an alkali metal-containing compound, an
    alkaline-earth metal and an alkaline-earth metal-containing compound relative to one equivalent of a benzylamine compound is 1 to 4 molar equivalent;
    a use amount of the benzylamine compound relative to 100 wt% of bis(aminomethyl)cyclohexane is 0.50 to 4.0 wt%;
    an isomerization reaction temperature of the isomerization step is 80 to 140°C; and
    the isomerization step and the distillation step are simultaneously carried out.

2. The method for producing the bis(aminomethyl)cyclohexane according to Claim 1, wherein the distillation step is carried out to separate a trans-isomer of 1,4-bis (aminomethyl)cyclohexane by distillation such that a content of the trans-isomer in the 1,4-bis(aminomethyl)cyclohexane obtained from the top part of the tower in the distillation step is 84% or more.

3. The method for producing the bis(aminomethyl)cyclohexane according to Claim 1 or 2, wherein the distillation step is carried out to separate a trans-isomer of 1,4-bis (aminomethyl)cyclohexane by distillation such that the content of the trans-isomer in the 1,4-bis(aminomethyl)cyclohexane obtained from the top part of the tower in the distillation step is 90% or more.

4. The method for producing the bis(aminomethyl)cyclohexane according to Claim 1, wherein the benzylamine compound is at least one selected from the group consisting of benzylamine, 3-methylbenzylamine, 4-methylbenzylamine, dibenzylamine, metaxylylenediamine and paraxylylenediamine.

5. The method for producing the bis(aminomethyl)cyclohexane according to Claim 1 or 4, wherein the alkali metal comprises metallic sodium.

6. The method for producing the bis(aminomethyl)cyclohexane according to any one of Claims 1 to 5, wherein the alkali metal-containing compound comprises at least one selected from the group consisting of an alkali metal hydride and an alkali metal amide.

7. The method for producing the bis(aminomethyl)cyclohexane according to Claim 6, wherein the alkali metal hydride comprises sodium hydride.

**8.** The method for producing the bis(aminomethyl)cyclohexane according to Claim 6, wherein the alkali metal amide comprises sodium amide.

**Patentansprüche**

**1.** Verfahren zur Erzeugung von Bis(aminomethyl)cyclohexan, enthaltend:

einen Isomerisierungsschritt zum Isomerisieren eines cis-Isomers von 1,4-Bis(aminomethyl)cyclohexan und/oder eines trans-Isomers von 1,3-Bis(aminomethyl)cyclohexan an einem Bodenteil eines Destillationsturmes, unter Erhalt eines trans-Isomers von 1,4-Bis(aminomethyl)cyclohexan und/oder eines cis-Isomers von 1,3-Bis(aminomethyl)cyclohexan, in der Gegenwart von zumindest einer Verbindung, ausgewählt aus der Gruppe, bestehend aus einem Alkalimetall, einer Alkalimetall-haltigen Verbindung, einem Erdalkalimetall und einer Erdalkalimetall-haltigen Verbindung, und einer Benzylaminverbindung, und

einen Destillationsschritt zum Trennen des trans-Isomers von 1,4-Bis(aminomethyl)cyclohexan und/oder des cis-Isomers von 1,3-Bis(aminomethyl)cyclohexan durch Destillation in einem oberen Teil des Destillationsturmes, worin

eine Verwendungsmenge der zumindest einen Verbindung, ausgewählt aus der Gruppe, bestehend aus einem Alkalimetall, Alkalimetall-haltigen Verbindung, Erdalkalimetall und Erdalkali-haltigen Verbindung in Bezug auf ein Äquivalent einer Benzylaminverbindung 1 bis 4 molare Äquivalente ist,

eine Verwendungsmenge der Benzylaminverbindung in Bezug auf 100 Gew.-% von Bis(aminomethyl)cyclohexan 0,50 bis 4,0 Gew.-% ist,

eine Isomerisationsreaktionstemperatur des Isomerisierungsschritts 80 bis 140°C ist und

der Isomerisierungsschritt und der Destillationsschritt gleichzeitig durchgeführt werden.

**2.** Verfahren zur Erzeugung von Bis(aminomethyl)cyclohexan gemäß Anspruch 1, worin der Destillationsschritt durchgeführt wird, zum Trennen eines trans-Isomers von 1,4-Bis(aminomethyl)cyclohexan durch Destillation, so dass ein Gehalt des trans-Isomers im 1,4-Bis(aminomethyl)cyclohexan, erhalten von dem oberen Teil des Turms in dem Destillationsschritt, 84 % oder mehr ist.

**3.** Verfahren zur Erzeugung von Bis(aminomethyl)cyclohexan gemäß Anspruch 1 oder 2, worin der Destillationsschritt durchgeführt wird, zum Trennen eines trans-Isomers von 1,4-Bis(aminomethyl)cyclohexan durch Destillation, so dass der Gehalt des trans-Isomers in 1,4-Bis(aminomethyl)cyclohexan, erhalten vom oberen Teil des Turmes im Destillationsschritt, 90 % oder mehr ist.

**4.** Verfahren zur Erzeugung von Bis(aminomethyl)cyclohexan gemäß Anspruch 1, worin die Benzylaminverbindung zumindest eine ist, ausgewählt aus der Gruppe, bestehend aus Benzylamin, 3-Methylbenzylamin, 4-Methylbenzylamin, Dibenzylamin, Metaxylylendiamin und Paraxylylendiamin.

**5.** Verfahren zur Erzeugung von Bis(aminomethyl)cyclohexan gemäß Anspruch 1 oder 4, worin das Alkalimetall metallisches Natrium enthält.

**6.** Verfahren zur Erzeugung von Bis(aminomethyl)cyclohexan gemäß einem der Ansprüche 1 bis 5, worin die Alkalimetall-haltige Verbindung zumindest eine enthält, ausgewählt aus der Gruppe, bestehend aus einem Alkalimetallhydrid und einem Alkalimetallamid.

**7.** Verfahren zur Erzeugung von Bis(aminomethyl)cyclohexan gemäß Anspruch 6, worin das Alkalimetallhydrid Natriumhydrid enthält.

**8.** Verfahren zur Erzeugung von Bis(aminomethyl)cyclohexan gemäß Anspruch 6, worin das Alkalimetallamid Natriumamid enthält.

**Revendications**

**1.** Procédé de production d'un bis(aminométhyl)cyclohexane comprenant :

une étape d'isomérisation consistant à isomériser un isomère cis de 1,4-bis(aminométhyl)cyclohexane et/ou d'un isomère trans de 1,3-bis(aminométhyl)cyclohexane au niveau d'une partie inférieure d'une tour de distillation pour obtenir un isomère trans de 1,4-bis(aminométhyl)cyclohexane et/ou un isomère cis de 1,3-bis(aminométhyl)cyclohexane, en présence d'au moins un composé choisi dans le groupe constitué par un métal alcalin, un composé contenant un métal alcalin, un métal alcalino-terreux et un composé contenant un métal alcalino-terreux, et un composé de benzylamine ; et

une étape de distillation consistant à séparer l'isomère trans de 1,4-bis(aminométhyl)cyclohexane et/ou l'isomère cis de 1,3-bis(aminométhyl)cyclohexane par distillation, dans une partie supérieure de la tour de distillation, dans lequel

une quantité d'utilisation du au moins un composé choisi dans le groupe constitué par un métal alcalin, un composé contenant un métal alcalin, un métal alcalino-terreux et un composé contenant un métal alcalino-terreux par rapport à un équivalent d'un composé de benzylamine est de 1 à 4 équivalents molaires ;

une quantité d'utilisation du composé de benzylamine par rapport à 100 % en poids de bis(aminométhyl)cyclohexane est de 0,50 à 4,0 % en poids ;

une température de réaction d'isomérisation de l'étape d'isomérisation est de 80 à 140°C ; et

l'étape d'isomérisation et l'étape de distillation sont effectuées simultanément.

2. Procédé de production du bis(aminométhyl)cyclohexane selon la revendication 1, dans lequel l'étape de distillation est réalisée pour séparer un isomère trans de 1,4-bis(aminométhyl)cyclohexane par distillation de telle sorte qu'une teneur de l'isomère trans dans le 1,4-bis(aminométhyl)cyclohexane obtenu à partir de la partie supérieure de la tour dans l'étape de distillation est de 84 % ou plus.

3. Procédé de production du bis(aminométhyl)cyclohexane selon la revendication 1 ou 2, dans lequel l'étape de distillation est réalisée pour séparer un isomère trans de 1,4-bis(aminométhyl)cyclohexane par distillation de telle sorte que la teneur en isomère trans dans le 1,4-bis(aminométhyl)cyclohexane obtenu à partir de la partie supérieure de la tour dans l'étape de distillation est de 90 % ou plus.

4. Procédé de production du bis(aminométhyl)cyclohexane selon la revendication 1, dans lequel le composé de benzylamine est au moins un choisi dans le groupe constitué par la benzylamine, la 3-méthylbenzylamine, la 4-méthylbenzylamine, la dibenzylamine, la métaxylylènediamine et la paraxylylènediamine.

5. Procédé de production du bis(aminométhyl)cyclohexane selon la revendication 1 ou 4, dans lequel le métal alcalin comprend du sodium métallique.

6. Procédé de production du bis(aminométhyl)cyclohexane selon l'une quelconque des revendications 1 à 5, dans lequel le composé contenant un métal alcalin comprend au moins un choisi dans le groupe constitué par un hydrure de métal alcalin et un amide de métal alcalin.

7. Procédé de production du bis(aminométhyl)cyclohexane selon la revendication 6, dans lequel l'hydrure de métal alcalin comprend de l'hydrure de sodium.

8. Procédé de production du bis(aminométhyl)cyclohexane selon la revendication 6, dans lequel l'amide de métal alcalin comprend de l'amide de sodium.

Fig.1

Fig.2

Fig.3

Fig.4

## REFERENCES CITED IN THE DESCRIPTION

**Patent documents cited in the description**

- WO 2009051114 A
- JP S5441804 B
- JP S50126638 B
- JP H10259167 B
- JP H10306066 B
- JP H11335335 B
- JP S623144 B
- JP H10330329 B
- JP 2011006382 A
- US 3344164 A
- JP 2013191881 A

**Non-patent literature cited in the description**

- *J. Polym. Sci. Part A-1,* 1972, vol. 10, 465
- *KOBUNSHI RONBUNSHU (Japanese Journal of Polymer Science and Technology),* 1979, vol. 65 (5), 305-310